(19) Europäisches Patentamt
European Patent Office
Office européen des brevets

(11) EP 0 646 369 B1

(12) **EUROPÄISCHE PATENTSCHRIFT**

(45) Veröffentlichungstag und Bekanntmachung des Hinweises auf die Patenterteilung:
**09.12.1998 Patentblatt 1998/50**

(51) Int. Cl.$^6$: **A61K 7/06**, A61K 7/32

(21) Anmeldenummer: **94115183.9**

(22) Anmeldetag: **27.09.1994**

(54) **Kosmetische Zubereitungen enthaltend 1-Hydroxy-2-Pyridone als Antischuppen- und desodorierende Mittel**

Cosmetic compositions comprising 1-hydroxy-2-pyridones as antidandruff and deodorant agents

Compositions cosmétiques contenant des 1-hydroxy-2-pyridones comme agents antipelliculaires et déodorants

(84) Benannte Vertragsstaaten:
**AT BE CH DE ES FR GB IT LI NL**

(30) Priorität: **05.10.1993 DE 4333893**

(43) Veröffentlichungstag der Anmeldung:
**05.04.1995 Patentblatt 1995/14**

(73) Patentinhaber: **Clariant GmbH**
**65929 Frankfurt am Main (DE)**

(72) Erfinder:
- **Hänel, Heinz, Dr.**
  **D-61440 Oberursel (DE)**
- **Simsch, Waltraud**
  **D-65779 Kelkheim (DE)**
- **Reng, Alwin K.**
  **D-65779 Kelkheim (DE)**

(56) Entgegenhaltungen:
**EP-A- 0 241 918** **EP-A- 0 386 900**
**US-A- 4 185 106**

- **MYCOSES, Bd.34, Nr.SUP1, 1991 Seiten 91 - 93 H. HÄNEL 'Therapie des seborrhoischen Ekzems mit einem antiphlogistisch wirksamen Antimykotikum.'**
- **DATABASE WPI Derwent Publications Ltd., London, GB; AN 84-033065 [06] & JP-A-58 222 010 (LION CORP.)**

EP 0 646 369 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Xerox (UK) Business Services
2.16.6/3.4

**Beschreibung**

Aus der DE-C-22 34 009 sind kosmetische Zubereitungen bekannt, die durch einen Gehalt an Verbindungen der allgemeinen Formel II

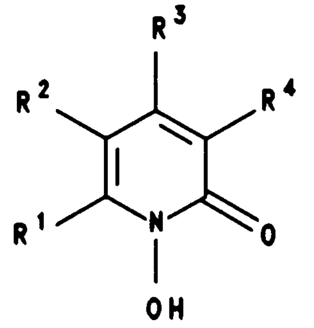

(II)

gekennzeichnet sind. In der obigen Formel II ist

$R^1$ u.a. Aryloxyalkyl mit Alkyl von 1 bis 4 Kohlenstoffatomen,
$R^2$ u.a. Wasserstoff, Alkyl mit 1 bis 4 Kohlenstoffatomen,
$R^3$ u.a. Wasserstoff, Alkyl mit 1 bis 4 Kohlenstoffatomen und
$R^4$ u.a. Wasserstoff, Alkyl mit 1 bis 4 Kohlenstoffatomen.

Der Rest $R^1$ wird spezifisch nur als Phenyloxymethyl angegeben.

Aus der EP-A-0 241 918 sind Verbindungen der Formel I bekannt

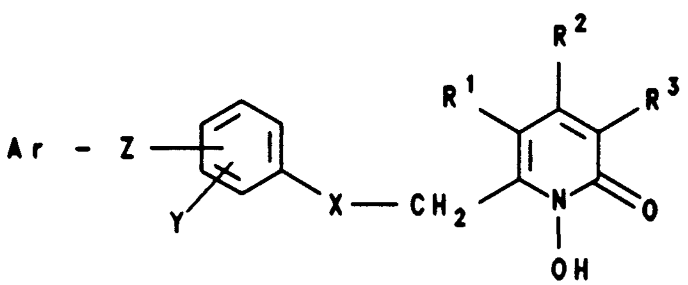

(I)

worin

| | |
|---|---|
| $R^1$, $R^2$ und $R^3$ | gleich oder verschieden sind und Wasserstoff oder Alkyl mit 1 bis 4 Kohlenstoffatomen bedeuten, wobei $R^1$ und $R^3$ vorzugsweise Wasserstoff sind und $R^2$ vorzugsweise Methyl ist, |
| X | S oder vorzugsweise O, |
| Y | Wasserstoff oder bis zu zwei Halogenatome, vorzugsweise Chlor und/oder Brom, |
| Z | eine Einfachbindung oder die zweiwertigen Reste O, S, $-CH_2-$, $-CH_2-O-$, $-O-CH_2-$, $-CH_2-S-$, $-S-CH_2-$ oder $-CH=CH-CH_2-O-$, |
| Ar | ein aromatisches Ringsystem ist mit bis zu zwei Ringen, das durch bis zu drei Reste aus der Gruppe Fluor, Chlor, Brom, Methoxy, $C_1$-$C_4$-Alkyl, Trifluormethyl und Trifluormethoxy substituiert sein kann. |

Als wichtiger Vertreter der durch die Formel I charakterisierten Verbindungsklasse wird u.a. 6-[4-(4-Chlor-phenoxy)-phenoxymethyl]-1-hydroxy-4-methyl-2-pyridon genannt. Diese Verbindung wird zur Vereinfachung nachfolgend als Rilopirox bezeichnet.

Die Verbindungen der allgemeinen Formel I besitzen topische antimykotische Eigenschaften mit einem breiten Wirkungsspektrum gegenüber pathogenen Pilzen, die sowohl die Haut als auch die Schleimhaut befallen, wie Hefen sowie Schimmelpilze. Diese Verbindungen können deshalb zur Bekämpfung von Infektionen durch diese Erreger in der Human- und Veterinärmedizin eingesetzt werden. Die Anwendung kann als freie Hydroxypyridone oder in Form ihrer physiologisch verträglichen Salze mit anorganischen oder organischen Basen in den für die Bekämpfung von Pilzen

üblichen Zubereitungsformen, wie Lösungen, Suspensionen, Cremes, Salben, Puder oder Suppositorien (Ovula) erfolgen.

Aus EP-A-0 241 918 ist lediglich die Verwendung der Verbindungen der Formel I als Arzneimittel bekannt.

Die Aufgabe der vorliegenden Erfindung besteht nun darin, kosmetische Zubereitungen, insbesondere Antischuppenmittel und desodorierende kosmetische Mittel zur Verfügung zu stellen.

Die vorliegende Erfindung betrifft kosmetische Zubereitungen, gekennzeichnet durch einen Gehalt an Verbindungen der allgemeinen Formel I

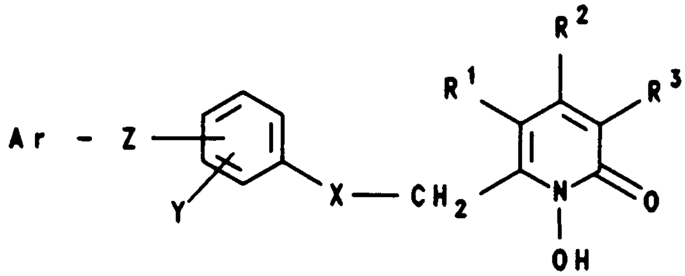

(I)

worin

| | |
|---|---|
| $R^1$, $R^2$ und $R^3$ | gleich oder verschieden sind und Wasserstoff oder Alkyl mit 1 bis 4 Kohlenstoffatomen bedeuten, wobei $R^1$ und $R^3$ vorzugsweise Wasserstoff sind und $R^2$ vorzugsweise Methyl ist, |
| X | S oder vorzugsweise O, |
| Y | Wasserstoff oder bis zu zwei Halogenatome, vorzugsweise Chlor und/oder Brom, |
| Z | eine Einfachbindung oder die zweiwertigen Reste O, S, $-CH_2-$, $-CH_2-O-$, $-O-CH_2-$, $-CH_2-S$, $-S-CH_2-$ oder $-CH = CH-CH_2-O-$, |
| Ar | ein aromatisches Ringsystem mit bis zu zwei Ringen, das durch bis zu drei Reste aus der Gruppe Fluor, Chlor, Brom, Methoxy, $C_1$-$C_4$-Alkyl, Trifluormethyl und Trifluormethoxy substituiert sein kann, |

und/oder deren Salzen.

Der Begriff aromatische Ringsysteme umfaßt Phenyl und kondensierte Systeme, wie Naphthyl, Tetrahydronaphthyl und Indenyl, sowie isolierte Systeme, die sich vom Biphenyl, Diphenylalkanen, Diphenylethern und Diphenylthioethern ableiten.

Diese kosmetischen Zubereitungen werden bevorzugt als Antischuppenmittel oder als desodorierendes kosmetisches Mittel verwendet.

Als bevorzugtes Beispiel für in den kosmetischen Zubereitungen erfindungsgemäß als Wirkstoff einsetzbare Substanz sei die folgende Verbindung genannt:

6-[4-(4-Chlor-phenoxy)-phenoxymethyl]-1-hydroxy-2-pyridon (genannt: Rilopirox)

Die obengenannten Verbindungen können sowohl in freier Form als auch als Salze eingesetzt werden.

Kommen organische Basen zur Anwendung, so werden vorzugsweise schwer flüchtige Basen eingesetzt, wie beispielsweise niedrigmolekulare Alkanolamine, wie z.B. Ethanolamin, Diethanolamin, N-Ethylethanolamin, N-Methyldiethanolamin, Triethanolamin, Diethylamino-ethanol, 2-Amino-2-methyl-n-propanol, Dimethylaminopropanol, 2-Amino-2-methyl-propandiol, Triisopropanolamin. Als weitere schwerer flüchtige Basen seien beispielsweise erwähnt Ethylendiamin. Hexamethylendiamin, Morpholin, Piperidin, Piperazin, Cyclohexylamin, Tributylamin, Dodecylamin, N,N-Dimethyl-dodecylamin, Stearylamin, Oleylamin, Benzylamin, Dibenzylamin, N-Ethyl-benzylamin, Dimethylstearylamin, N-Methylmorpholin, N-Methylpiperazin, 4-Methylcyclohexylamin, N-Hydroxyethyl-morpholin. Auch die Salze quartärer Ammoniumhydroxide, wie z.B. Trimethylbenzylammoniumhydroxid, Tetramethylammoniumhydroxid oder Tetraethylammoniumhydroxid können verwendet werden, ferner Guanidin und seine Abkömmlinge, insbesondere seine Alkylierungsprodukte. Es ist jedoch auch möglich, als Salzbildner beispielsweise niedrigmolekulare Alkylamine, wie z.B. Methylamin, Ethylamin oder Triethylamin einzusetzen. Auch Salze mit anorganischen Kationen, wie beispielsweise Alkalisalze, insbesondere Natrium-, Kalium- oder Ammonium-Salze, Erdalkalisalze, wie insbesondere das Magnesium- oder Calciumsalz, sowie Salze mit 2 bis 4-wertigen Kationen, wie beispielsweise das Zink-, Aluminium- oder Zirkon-Salz kommen für die erfindungsgemäß einzusetzenden Verbindungen in Betracht.

Die in den kosmetischen Zubereitungen einzusetzenden Wirkstoffe der oben wiedergegebenen Formel können beispielsweise nach Verfahren hergestellt werden, wie sie in dem US-Patent 4 797 409 beschrieben werden. Die Herstellung der obengenannten Salze erfolgt in bekannter Weise durch Zusammengeben von vorzugsweise äquimolaren Mengen der salzbildenden Komponenten.

Für den erfinderungsgemäßen Einsatz der genannten Verbindungen kommen die unterschiedlichsten kosmetischen Zubereitungen in Betracht, insbesondere Shampoos. Als Beispiele für weitere erfindungsgemäß in Betracht kommende Zubereitungen seien folgende Haarpflege- und Frisiermittel erwähnt: Haarspülmittel, Haarkuren, Haarregeneriermittel, Haarwässer, Wasserwellotionen, Haarsprays, Frisiercremes, Frisiergels, Haaröle, Haarpomaden oder Haarbrillantinen. Es handelt sich demnach immer um Zubereitungen, die je nach ihrem eigentlichen Anwendungszweck für kürzere oder längere Zeit auf das Haar und die Kopfhaut aufgebracht werden. Durch die Zugabe der erfindungsgemäßen Verbindungen wird dann eine gleichzeitige Schuppenbehandlung bewirkt. Es ist jedoch auch möglich, Zubereitungen herzustellen, die in erster Linie oder ausschließlich dem Ziel einer Schuppenbeseitigung dienen.

Werden die erfindungsgemäßen Antischuppenzubereitungen als Shampoos dargeboten, so können diese klarflüssig, opakflüssig (mit Perlglanzeffekt), cremeförmig, gelartig, aber auch pulverförmig oder in Tablettenform, sowie als Aerosole konditioniert sein. Die diesen Shampoos zugrunde liegenden Waschrohstoffe können anionischer, kationischer, nichtionischer und amphoterer Natur sein und auch in Kombinationen dieser Stoffe vorliegen.

Als Beispiele für derartige anionische waschaktive Substanzen seien genannt: $C_{10}$-$C_{20}$-Alkyl- und Alkylen-carboxylate, Alkylethercarboxylate, Fettalkoholsulfate, Fettalkoholethersulfate, Alkylolamidsulfate und -sulfonate, Fettsäurealkylolamidpolyglykolethersulfate, Alkansulfonate und Hydroxyalkansulfonate, Olefinsulfonate, Acylester von Isethionaten, $\alpha$-Sulfofettsäureester, Alkylbenzolsulfonate, Alkylphenolglykolethersulfonate, Sulfosuccinate, Sulfobernsteinsäurehalbester und -diester, Fettalkoholetherphosphate, Eiweiß-Fettsäure-Kondensationsprodukte, Alkylmonoglyceridsulfate und -sulfonate, Alkylglyceridethersulfonate, Fettsäuremethyltauride, Fettsäuresarkosinate, Sulforicinoleate. Diese Verbindungen und deren Mischungen werden in Form ihrer wasserlöslichen oder in Wasserdispergierbaren Salze benutzt, beispielsweise der Natrium-, Kalium-, Magnesium-, Ammonium-, Mono-, Di- und Triethanolammonium- sowie analogen Alkylammonium-Salze.

Geeignete kationische Tenside sind beispielsweise quartäre Ammonium-Salze wie Di-($C_{10}$-$C_{24}$-Alkyl)-dimethyl-ammonium-chlorid oder -bromid, vorzugsweise Di-($C_{12}$-$C_{18}$-Alkyl)-dimethyl-ammonium-chlorid oder -bromid; $C_{10}$-$C_{24}$-Alkyl-dimethyl-ethylammonium-chlorid oder -bromid; $C_{10}$-$C_{24}$-Alkyl-trimethyl-ammonium-chlorid oder -bromid, vorzugsweise Cetyl-trimethyl-ammonium-chlorid oder -bromid und $C_{20}$-$C_{22}$-Alkyl-trimethyl-ammonium-chlorid oder - bromid; $C_{10}$-$C_{24}$-Alkyl-dimethylbenzyl-ammonium-chlorid oder -bromid, vorzugsweise $C_{12}$-$C_{18}$-Alkyl-dimethylbenzyl-ammonium-chlorid; N-($C_{10}$-$C_{18}$-Alkyl)-pyridinium-chlorid oder -bromid, vorzugsweise N-($C_{12}$-$C_{16}$-Alkyl)-pyridinium-chlorid oder -bromid; N-($C_{10}$-$C_{18}$-Alkyl)-isochinolinium-chlorid, -bromid oder -monoalkylsulfat; N-($C_{12}$-$C_{18}$-Alkyloylcolaminoformylmethyl)pyridinium-chlorid; N-($C_{12}$-$C_{18}$-Alkyl)-N-methyl-morpholinium-chlorid, -bromid oder -monoalkylsulfat; N-($C_{12}$-$C_{18}$-Alkyl)-N-ethyl-morpholinium-chlorid, - bromid oder -monoalkylsulfat; $C_{16}$-$C_{18}$-Alkyl-pentaoxethyl-ammonium-chlorid; Diisobutyl-phenoxyethoxyethyldimethylbenzylammonium-chlorid; Salze des N,N-Diethylaminoethylstearylamids und -oleylamids mit Salzsäure, Essigsäure, Milchsäure, Zitronensäure, Phosphorsäure; N-Acyl-amidoethyl-N,N-diethyl-N-methyl-ammoniumchlorid, -bromid oder -monoalkylsulfat und N-Acylaminoethyl-N,N-diethyl-N-benzyl-ammonium-chlorid, -bromid oder -monoalkylsulfat, wobei Acyl vorzugsweise für Stearyl oder Oleyl steht.

Als nichtionische Tenside, die als waschaktive Substanzen eingesetzt werden können, kommen beispielsweise in Betracht: Fettalkoholethoxylate (Alkylpolyethylenglykole); Alkylphenolpolyethylenglykole; Alkylmercaptanpolyethylenglykole; Fettaminethoxylate (Alkylaminopolyethylenglykole); Fettsäureethoxylate (Acylpolyethylenglykole); Polypropylenglykolethoxylate (Pluronic); Fettsäurealkylolamide (Fettsäureamidpolyethylenglykole); Saccharoseester; Sorbitester und der Polyglykolether.

Beispiele für den Shampoos zusetzbare Amphotenside sind: N-($C_{12}$-$C_{18}$-Alkyl)-$\beta$-aminopropionate und N-($C_{12}$-$C_{18}$-Alkyl)-$\beta$-iminodipropionate als Alkali- und Mono-, Di- und Trialkylammonium-Salze; N-Acylamidoalkyl-N,N-dimethylacetobetain, vorzugsweise N-($C_8$-$C_{18}$-Acyl)amidopropyl-N,N-dimethylacetobetain; $C_{12}$-$C_{18}$-Alkyl-dimethyl-sulfopropyl-betain; Amphotenside auf Basis Imidazolin (Handelsname: Miranol®, Steinapon®), vorzugsweise das Natrium-Salz des 1-($\beta$-Carboxy-methyloxyethyl)-1-(carboxymethyl)-2-laurylimidazoliniums; Aminoxid, z.B. $C_{12}$-$C_{18}$-Alkyldimethyl-aminoxid, Fettsäureamidoalkyl-dimethylaminoxid.

Die erfindungsgemäßen Zubereitungen können außerdem weitere in der Kosmetik gebräuchliche Zusätze enthalten, z.B. Riechstoffe, Farbstoffe, auch solche, die gleichzeitig die Haare färben oder tönen, Lösungsmittel, Trübungsmittel und Perlglanzmittel, beispielsweise Ester von Fettsäuren mit Polyolen, Magnesium- und Zink-Salze von Fettsäuren, Dispersionen auf Basis Mischpolymerer, Verdickungsmittel wie Natrium-, Kalium-, Ammoniumchlorid, Natriumsulfat, Fettsäurealkylolamide, Cellulosederivate, natürliche Gummen, ferner Pflanzenextrakte, Eiweißderivate wie Gelatine, Collagenhydrolysate, Polypeptide auf natürlicher und synthetischer Basis, Eigelb, Lecithin, Lanolin und Lanolinderivate, Fette, Öle, Fettalkohole, Silicone, desodorierende Mittel, antimikrobiell wirksame Substanzen, antiseborrhoeisch wirksame Substanzen, Stoffe mit keratolytischer und keratoplastischer Wirkung, wie beispielsweise Schwefel, Salicylsäure, Enzyme.

Zur Herstellung der kosmetischen Zubereitungen wird die Wirksubstanz bei einer Temperatur im Bereich von 20 - 60°C, vorzugsweise bei Raumtemperatur, in dem zum Einsatz kommenden Tensid unter Rühren gelöst. Anschließend

werden die einzelnen gebräuchlichen Zusätze zugegeben.
Im Fall von alkoholhaltigen kosmetischen Zubereitungen wird die Wirksubstanz in dem Alkohol bei einer Temperatur im Bereich von 20 - 40°C, vorzugsweise bei Raumtemperatur, gelöst. Anschließend werden die einzelnen gebräuchlichen Zusätze zugegeben.
Im Fall von Haarspülmitteln bzw. O/W-Emulsionen wird der Wirkstoff in der Fettphase, d.h. zusammen mit dem Öl und dem Emulgator, bei einer Temperatur im Bereich von 70 bis 90°C, vorzugsweise 75°C, gelöst. Anschließend wird heißes Wasser zugegeben und die Emulsion so lange gerührt, bis sie erkaltet ist.

Die Herstellung der Shampoos erfolgt in an sich bekannter Weise durch Zusammengeben der einzelnen Komponenten und eine - soweit erforderlich - der jeweiligen Zubereitungsart angepaßte Weiterverarbeitung. Einige dieser vielfältigen möglichen Zubereitungsformen werden in den Ausführungsbeispielen beispielhaft beschrieben.

Als weitere haarkosmetische Zubereitungen, in denen die 1-Hydroxi-2-pyridone erfindungsgemäß zur Anwendung kommen können, seien beispielsweise genannt: Haarspülmittel (sogenannte Hair rinses), Haarkuren und Haarregeneriermittel, die nach einer gewissen Zeit vom Haar abgespült werden oder je nach Formulierung auch auf dem Haar verbleiben können. Diese Präparate enthalten unter anderem Substanzen aus der Gruppe der obenerwähnten kationische Tensiden, die am Haar eine avivierende und antistatische Eigenschaft entfalten.

Die erfindungsgemäßen Antischuppenzubereitungen können auch in Form von wäßrigen und wäßrig-alkoholischen Haarwässern, Wasserwellotionen (Haarfestiger), auch solcher in Gelform, und in Aerosolform als Haarspray, sowie in Form von Haarpflege- und Frisiercremes und -gels dargeboten werden. Als Alkohole werden vorzugsweise Ethanol und Isopropanol eingesetzt.

Beispiele für Harze mit haarfestigender und frisurenfestigender Wirkung, die in einer Konzentration von 0,5 bis 6 Gew.-%, vorzugsweise 1 bis 3 Gew.-%, in den entsprechenden Zubereitungen (Haarfestiger, Haarspray) enthalten sein können, sind: Schellack und -derivate, Umsetzungsprodukte von Kolophonium mit Acrylsäure, Poly-N-vinylpyrrolidon und alkylsubstituierte Poly-N-vinylpyrrolidon, Poly-N-vinyl-N-alkyl-acetamid, Polyvinylacetat und partiell verseiftes Polyvinylacetat, Polyvinylalkohol, Alkylester der Acrylsäure, Mischpolymerisate aus Vinylacetat und N-Vinyl-N-alkylacetamid, Mischpolymerisate aus Vinylacetat und N-Vinylpyrrolidon, Umsetzungsprodukte aus Mischpolymerisaten aus Vinylacetat und Acrylsäure bzw. Crotonsäure mit organischen Basen, Mischpolymerisate aus Vinylacetat und Maleinsäurehalbester, Mischpolymerisate aus Vinylacetat, Vinylpivalat und Crotonsäure, Mischpolymerisate aus Fettsäurevinylestern und (Meth)acrylsäure, Mischpolymerisate aus (Meth)acrylsäureestern und N-Vinylpyrrolidon, Mischpolymerisate aus Acrylsäureestern und (Meth)acrylsäure, Alkylester aus Mischpolymerisaten aus Methylvinylether und Maleinsäureanhydrid, Alkylester aus Mischpolymerisaten aus Olefinen und Maleinsäureanhydrid, Polyvinylacetale und Polyvinylbutyrale, Dimethylhydantoin-Formaldehyd-Kondensate, Cyclohexanon-Formaldehyd-Harze, Phthalatharze, Eiweißhydrolysate und Eiweißderivate, Stärke- und Cellulose-derivate, die auch kationische Gruppen enthalten können, sowie weitere Filmbildner mit quartären Gruppen wie Umsetzungsprodukte von Mischpolymeren aus Alkyl(meth)acrylaten und Dimethylaminoethyl(meth)acrylat mit Alkylierungsmitteln, ferner quartäre Copolymere aus N-Vinylpyrrolidon und Dialkylaminoalkyl(meth)acrylaten.

Es ist weiterhin möglich, die erfindungsgemäß einsetzbaren Verbindungen in wasserfreien öligen Zubereitungen wie Haaröl, Haarpomade, Haarbrillantine einzuarbeiten.

Auch die Herstellung aller dieser Zubereitungen erfolgt - wie bereits beim Shampoo erwähnt - in an sich bekannter Weise unter Zugabe des erfindungsgemäß eingesetzten Wirkstoffs. Die erfindungsgemäßen Antischuppenzubereitungen können von den obengenannten 1-Hydroxi-2-pyridonen eine Verbindung oder auch mehrere in Kombination enthalten.

In die erfindungsgemäßen Zubereitungen wird der Antischuppenwirkstoff in Mengen eingearbeitet, die üblicherweise zwischen etwa 0,05 und etwa 10 % liegen. Innerhalb dieses Bereiches richten sich die Konzentrationen der speziellen Zubereitungen nach ihrem Anwendungszweck. Bestimmte Zubereitungsformen, wie z.B. Konzentrate, die vor ihrer Anwendung zu verdünnen sind, können auch erheblich höhere Konzentrationen aufweisen.

Handelt es sich um Zubereitungen, die auf dem Haar verbleiben, wie beispielsweise Haarwasser, Haarfestiger, Cremes usw., so wird man niedrigere Konzentrationen einsetzen, beispielsweise von ca. 0,01 bis ca. 1 %, vorzugsweise 0,1 bis 0,5 %. In höheren Konzentrationen werden sie zweckmäßigerweise dann zur Anwendung kommen, wenn es sich um kosmetische Zubereitungen handelt, die, gegebenenfalls nach Verdünnung, nur kurze Zeit auf Haar und Kopfhaut einwirken, wie beispielsweise Shampoos oder Haarspülmittel. In diesen Fällen können z.B. Konzentrationen von etwa 0,2 bis etwa 10 %, vorzugsweise etwa 0,5 bis etwa 2 %, zweckmäßig sein.

Es ist bekannt, daß 1-Hydroxi-2-pyridinthione und deren Salze, insbesondere das Zinksalz, gegen Schuppen wirksam sind. Es war nicht zu erwarten, daß auch die schwefelfreien Verbindungen erfindungsgemäß eine ausgezeichnete Antischuppenwirkung zeigen.

Die erfindungsgemäße Verwendung der genannten 1-Hydroxi-2-pyridonen zeichnet sich insbesondere gegenüber dem erwähnten Stand der Technik durch zahlreiche Vorteile aus.

Herauszuheben sind:

- geringe Einsatzkonzentrationen
- breites antimikrobielles Wirkungsspektrum
- geringe Toxizität.

Die nachstehenden Beispiele erläutern die vorliegende Erfindung. Die Mengenangaben sind auf das Gewicht bezogen.

Beispiel 1 - Cremeshampoo

| | |
|---|---|
| Rilopirox | 0,2 % |
| Fettsäuremethyltaurid-Natriumsalz (® Hostapon CT-Teig, Hoechst AG) | 70 % |
| Fettsäuremethylisethionat-Natriumsalz (® Hostapon SCID, Hoechst AG) | 5 % |
| Palmkernfettsäuresarkosid (® Medialan LD) | 5 % |
| Wasser | ad 100 % |

Beispiel 2 - Antischuppenshampoo

| | |
|---|---|
| Rilopirox | 0,3 % |
| Fettsäuremonoethanolamidpolyglykolether (® Genagen CA-050, Hoechst AG) | 5 % |
| Alkylethersulfat-Natriumsalz (® Genapol LRO fl., Hoechst AG) | 35,0 % |
| Acylaminopolyglykolethersulfat-Magnesiumsalz (® Genapol AMG, Hoechst AG) | 10,0 % |
| Alkylamidopropyl-Betain (® Genagen CAB, Hoechst AG) | 8,0 % |
| Konsistenzgeber (® Genapol L-3, Hoechst AG) | 2,0 % |
| Natriumchlorid | 0,7 % |
| Wasser | ad 100 % |

Beispiel 3 - "3 in 1-Shampoo"

| | |
|---|---|
| Rilopirox | 0,2 % |
| Alkylethersulfat-natriumsalz (® Genapol LRO. fl., Hoechst AG) | 35,0 % |
| Acylaminopolyglykolethersulfat-Magnesiumsalz (® Genapol AMG, Hoechst AG) | 5,0 % |
| Fettsäureglutamat-natriumsalz (® Hostapon KCG, Hoechst AG) | 5,0 % |
| Fettsäure-Protein-Kondensat (® Hostapon SCHC, Hoechst AG) | 5,0 % |
| Silikonöl (® Belsil DNC 6032, Wacker Chemie) | 2,0 % |
| D-Panthenol | 1,0 % |
| Konsistenzgeber (® Genapol L-3, Hoechst AG) | 2,5 % |
| Alkylamidopropyl-Betain (® Genagen CAB, Hoechst AG) | 8,0 % |
| Natriumchlorid | 2,0 % |
| Wasser | ad 100 % |

Beispiel 4 - Shampookonzentrat

| | |
|---|---|
| Rilopirox | 0,2 % |
| Alkylethersulfat-Natriumsalz (® Genapol LRO Paste, Hoechst AG) | 20,0 % |
| Alkylamidopropyl-Betain (® Genapol CAB, Hoechst AG) | 33,0 % |
| Fettsäuremonoethanolamidpolyglykolether (® Genagen CA-050, Hoechst AG) | 5,0 % |
| Fettsäure-Protein-Kondensat (® Hostapon SCHCP, Hoechst AG) | 5,5 % |
| Konsistenzgeber (® Genapol L-3, Hoechst AG) | 3,0 % |
| Wasser | ad 100 % |

Beispiel 5 - flüssige Seife

| | |
|---|---|
| Rilopirox | 0,1 % |
| Alkylethersulfat-Natriumsalz (® Genapol LRO, Hoechst AG) | 40,0 % |
| sek. n-Alkylsulfonat-Natriumsalz (® Hostapur SAS, Hoechst AG) | 8,0 % |
| Konsistenzgeber (® Genapol L-3, Hoechst AG) | 3,0 % |
| Natriumchlorid | 1,5 % |
| Wasser | ad 100 % |

Beispiel 6 - Deoseife

| | |
|---|---|
| Rilopirox | 0,2 % |
| Grundseife | 99,8 % |

Beispiel 7 - Roll-on Deo

| | |
|---|---|
| Rilopirox | 0,1 % |
| Hydroxyethylcelluloseether (® Tylose H 10000, Hoechst AG) | 0,7 % |
| Ethanol | 40,0 % |
| 1,2-Propylenglykol | 5,0 % |
| Lösungsvermittler (® Cremophor RH 455, BASF AG) | 0,5 % |
| Wasser | 53,0 % |

Beispiel 8 - Deodorant

| | |
|---|---|
| Rilopirox | 0,15 % |

(fortgesetzt)

| | |
|---|---|
| Ethanol | 70,0 % |
| Rückfettungsmittel (® Softigen 767, Chem. Werke Hüls) | 0,5 % |
| Parfümöl | 0,5 % |
| Allantoin | 0,1 % |
| Wasser | ad 100 % |

Beispiel 9 - Intimwaschmittel

| | |
|---|---|
| Rilopirox | 0,1 % |
| Acylaminopolyglykolethersulfat-Magnesiumsalz (® Genapol AMG, Hoechst AG) | 40,0 % |
| Konsistenzgeber (Glucamate DOE 120, Amercol) | 3,0 % |
| Wasser | ad 100 % |

Beispiel 10 - Haarnachspülmittel

| | |
|---|---|
| Rilopirox | 0,2 % |
| Cetylalkohol | 2,5 % |
| Paraffinöl | 1,5 % |
| Phosphorsäureesterverbindung (® Hostaphat KL340N, Hoechst AG) | 1,5 % |
| Alkylpolyethoxyammoniumlactat (® Genamin KSL, Hoechst AG) | 7,0 % |
| Hydroxyethylcelluloseether (® Tylose, Hoechst AG) | 0,6 % |
| Wasser | ad 100 % |

Beispiel 11 - Haarwasser

| | |
|---|---|
| Rilopirox | 0,05 % |
| Fettsäurepolyglykolether (® Emulsogen EL, Hoechst AG) | 0,6 % |
| Ethanol | 40,0 % |
| Alkylpolyethoxyammoniumlactat (® Genamin KSL, Hoechst AG) | 0,3 % |
| D-Panthenol | 0,5 % |
| Wasser | ad 100 % |

Beispiel 12 - Haarfestiger

| | |
|---|---|
| Rilopirox | 0,05 % |

(fortgesetzt)

| | |
|---|---|
| Isopropanol | 40,0 % |
| Polyethylenglykol ($\overline{M}_w$ = 400) | 0,5 % |
| Alkylpolyethoxyammoniumlactat (® Genamin KSL, Hoechst AG) | 0,7 % |
| Rückfettungsmittel | 0,6 % |
| Polyvinylpyrrolidon (® Luviskol, BASF AG) | 5,5 % |
| Wasser | ad 100 % |

Beispiel 13 - Gesichtswasser

| | |
|---|---|
| Rilopirox | 0,05 % |
| Ethanol | 30,0 % |
| Rückfettungsmittel | 0,2 % |
| D-Panthenol | 0,5 % |
| 1,2-Propylenglykol | 5,0 % |
| Allantoin | 0,1 % |
| Extrapon Hamamelis | 5,0 % |
| Wasser | ad 100 % |

Beispiel 14 - Öl-in-Wasser-Creme

| | |
|---|---|
| Rilopirox | 0,1 % |
| 1,2-Propylenglykol | 10,0 % |
| Öl-in-Wasser-Emulgator (® Hostacerin CG, Hoechst AG) | 12,0 % |
| Ölkomponente (® Entanol G, Henkel KG & A) | 8,0 % |
| Paraffinöl | 5,0 % |
| Isopropylisostearat | 5,0 % |
| Konsistenzgeber (® Carbopol 940, Goodrich) | 0,3 % |
| Natronlauge (10 %ig) | 0,4 % |
| Wasser | ad 100 % |

## Patentansprüche

1. Kosmetische Zubereitungen, gekennzeichnet durch einen Gehalt an Verbindungen der allgemeinen Formel I

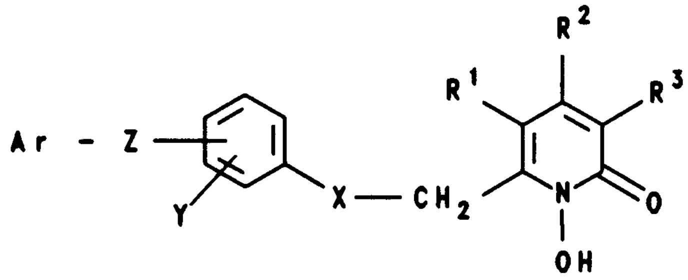

(I)

worin

| | |
|---|---|
| $R^1$, $R^2$ und $R^3$ | gleich oder verschieden sind und Wasserstoff oder Alkyl mit 1 bis 4 Kohlenstoffatomen bedeuten, wobei $R^1$ und $R^3$ vorzugsweise Wasserstoff sind und $R^2$ vorzugsweise Methyl ist, |
| X | S oder O, |
| Y | Wasserstoff oder bis zu zwei Halogenatome, vorzugsweise Chlor und/oder Brom, |
| Z | eine Einfachbindung oder die zweiwertigen Reste O, S, - $CH_2$-, -$CH_2$-O-, -O-$CH_2$-, -$CH_2$-S-, -S-$CH_2$- oder -CH = CH-$CH_2$-O-, |
| Ar | ein aromatisches Ringsystem mit bis zu zwei Ringen, das durch bis zu drei Resten aus der Gruppe Fluor, Chlor, Brom, Methoxy, $C_1$-$C_4$-Alkyl, Trifluormethyl und Trifluormethoxy substituiert sein kann, |

und/oder deren Salzen.

2. Kosmetische Zubereitungen, gekennzeichnet durch einen Gehalt an 6-[4-(4-Chlor-phenoxy)-phenoxymethyl]-1-hydroxy-4-methyl-2-pyridon.

3. Verwendung der kosmetischen Zubereitung nach Anspruch 1 oder 2 als Antischuppenmittel.

4. Verwendung der kosmetischen Zubereitung nach Anspruch 1 oder 2 als desodorierendes kosmetisches Mittel.

## Claims

1. A cosmetic preparation which has a content of compounds of the formula I

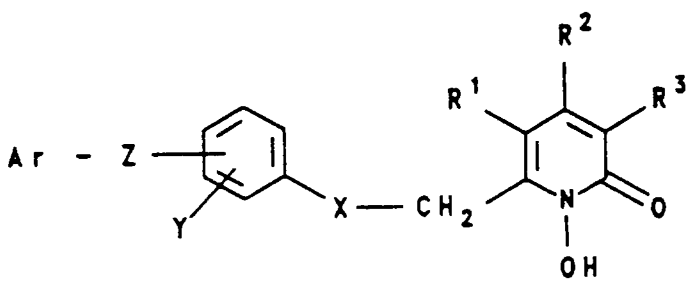

(I)

in which

| | |
|---|---|
| $R^1$, $R^2$ and $R^3$ | are identical or different and are hydrogen or alkyl with 1 to 4 carbon atoms, where $R^1$ and $R^3$ are preferably hydrogen and $R^2$ is preferably methyl, |
| X | is S or O, |
| Y | is hydrogen or up to two halogen atoms, preferably chlorine and/or bromine, |
| Z | is a single bond or the divalent radicals O, S, -$CH_2$-, -$CH_2$-O-, -O-$CH_2$-, -$CH_2$-S-, -S-$CH_2$- or -CH=CH-$CH_2$-O-, |
| Ar | is an aromatic ring system which has up to two rings and which can be substituted by up to three radicals from the group comprising fluorine, chlorine, bromine, methoxy, $C_1$-$C_4$-alkyl, trifluorome- |

thyl and trifluoromethoxy,

and/or their salts.

2. A cosmetic preparation which has a content of 6-[4-(4-chlorophenoxy)phenoxymethyl]-1-hydroxy-4-methyl- 2-pyridone.

3. The use of the cosmetic preparation as claimed in claim 1 or 2 as antidandruff composition.

4. The use of the cosmetic preparation as claimed in claim 1 or 2 as deodorant cosmetic composition.

**Revendications**

1. Préparations cosmétiques, caractérisées par une teneur en composés de formule générale I

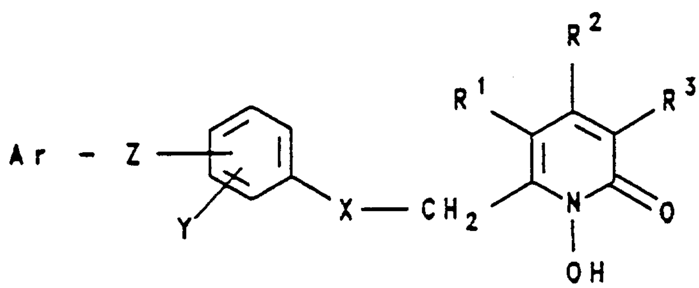

(I)

dans laquelle

| | |
|---|---|
| $R^1$, $R^2$ et $R^3$ | sont identiques ou différents et représentent un atome d'hydrogène ou un groupe alkyle avec 1 à 4 atomes de carbone, $R^1$ et $R^3$ étant de préférence un atome d'hydrogène et $R^2$ de préférence un groupe méthyle, |
| X | représente un atome de soufre ou un atome d'oxygène, |
| Y | représente un atome d'hydrogène ou jusqu'à deux atomes d'halogène, de préférence le chlore et/ou le brome, |
| Z | représente une liaison simple ou les restes bivalents O, S, $-CH_2-$, $-CH_2-O-$, $-O-CH_2-$, $-CH_2-S-$, $-S-CH_2-$ ou $-CH=CH-CH_2-O-$, |
| Ar | représente un système cyclique aromatique avec jusqu'à deux cycles qui peut être substitué par jusqu'à trois restes pris dans le groupe comportant des atomes de fluor, de chlore, de brome, des groupes méthoxy, alkyle en $C_1$-$C_4$, trifluorométhyle et trifluorométhoxy, |

et/ou leurs sels.

2. Préparations cosmétiques caractérisées par une teneur en 6-[4-(4-chloro-phénoxy)-phénoxyméthyl]-1-hydroxy-4-méthyl-2-pyridone.

3. Utilisation de la préparation cosmétique selon la revendication 1 ou 2 en tant que produit anti-pelliculaire.

4. Utilisation de la préparation cosmétique selon la revendication 1 ou 2 en tant que produit cosmétique déodorant.